# EUROPEAN PATENT APPLICATION

(11) **EP 4 105 315 A1**
(43) Date of publication of application: **21.12.2022**
(21) Application number: 22178664.3
(22) Date of filing: 13.06.2022
(51) Int. Cl.: C12M 1/33, C12M 1/00

(54) **ORGANIC WASTE PRE-TREATMENT SYSTEM FOR SUCCESSIVE ANAEROBIC DIGESTION AND RELATED PRE-TREATMENT METHOD**

(30) Priority: 15.06.2021 IT 202100015524
(71) Applicant: Calabra Maceri e Servizi S.p.A., 87036 Rende (CS) (IT); Biotech Impianti S.r.l, 87100 Cosenza (IT); Waste To Methane S.r.l., 87036 Rende (CS) (IT)
(72) Inventor: ZANARDI, Giuseppe, 87036 Rende, COSENZA (IT); BIANCO, Micaela, 87036 Rende, COSENZA (IT); D'AMICO, Francesco, 87036 Rende, COSENZA (IT)
(74) Representative: Mozzi, Matteo

(57) **Abstract**

An organic waste pre-treatment system (100) for successive anaerobic digestion, comprising:
- a first organic waste pre-treatment station (10) configured to open packages of organic waste and shred the organic waste into particles, said first organic waste pre-treatment station (10) being configured to convey the waste particles to a successive processing;
- at least a second organic waste pre-treatment station (20) configured to receive the organic waste particles conveyed from the first organic waste pre-treatment station (10), said at least a second organic waste pre-treatment station (20) being configured to separate such particles into sub-particles of a moist putrescible organic component and into sub-particles of a non-putrescible waste material component, said at least a second organic waste pre-treatment station (20) being configured to convey a compound of sub-particles of the moist putrescible organic component having a smaller size than a set sifting size to a successive processing;
- a third pre-treatment station (30) configured to accumulate the compound of sub-particles of the moist putrescible organic component having a smaller size than a set sifting size;
- a feeding device (40) configured to transfer the compound from the third organic waste pre-treatment station (30) to anaerobic digesters.

## Description

### . Field of the invention

. The present invention relates to the field of the anaerobic digestion of organic waste, and in particular to an organic waste pre-treatment system for successive anaerobic digestion and related organic waste pre-treatment method.

### . Background art

. The anaerobic digestion of organic waste is an established industrial technology which allows material and energy to be recovered, and is increasingly implemented to manage household organic waste (waste from the preparation and consumption of food) and biodegradable waste in general.

. It is a biological process carried out in the absence of oxygen which results in the microbial degradation of the biodegradable organic substance with production of a gas, so-called biogas, essentially composed of methane (in percentages generally comprised between 50 and 80% by volume) and carbon dioxide, employed for producing (electrical or thermal) energy or, following purification, bio-methane for motor propulsion or for transfer to distribution networks.

. Anaerobic digestion also generates an important flow of biomass (called digestate) resulting from the biological process which can be used as soil improver as is or after possible aerobic maturation.

. The pre-treatment of organic waste to be sent to successive anaerobic digestion is a very important step because it allows obtaining benefits for the successive anaerobic digestion such as, on the one hand, the separation and waste of non-fermentable materials present in the waste, such as plastics, metals, fabrics ad glass and, on the other hand, the reduction in size of the particles forming the pre-treated organic waste (ingestate) in order to promote the microbial attack and speed up fermentation.

. Nowadays, the pre-treatment of organic waste conventionally is carried out by using a system comprising a shredder consisting of a metal tank open at the top on the bottom of which is placed a cylindrical member (rotor) provided with metal teeth which, turning against a fixed comb, tears the plastic bags and breaks up the material, thus allowing the same to pass below the tank only if reduced to dimensions that are less than the distance between rotor and fixed comb.

. The system, downstream of the shredder, comprises a size separator generally consisting of a rotating drum sieve, which consists of a rotating metal cylinder which walls are provided with holes having diameter between 50 mm and 80 mm, or of a rotating disc or star sieve with opening having similar dimensions, which allow the organic material, conventionally received by means of a conveyor screw, to pass while retaining most of the plastic bags and other coarse materials (fabrics, cans, pieces of wood) having larger dimensions than the holes and that thus are sent for disposal or treatments other than anaerobic digestion.

. Lastly, the pre-treatment system comprises an accumulating and dosing tank (usually called bunker) of the sieved organic material to be fed to the digesters, arranged downstream of the sieve or size separator, the presence of which is due to the fact that the delivery of the organic waste and the processing thereof usually occur during regular work shifts on working days, while the anaerobic digestion is a biological process that requires a constant and continuous feeding 24 hours a day, 7 days a week.

. This accumulating and dosing tank conventionally has a volume such as to contain the substrate of organic material for at least one and a half working days (weekend) and is provided with a movable bottom with metal plates which slowly slide towards the unloading side, thus supplying a conveyor belt or a conveyor screw that carries the pre-treated organic material to an anaerobic digester.

. In order to feed an anaerobic digester, the pre-treated organic material has to be carried to a higher height than the one of the level of the substrate in the digesters, and from said height has to be introduced therein by means of a conveyor screw tilted downwards that introduces the material below the filling level.

. Thereby, the introduction conveyor screw serves as hydraulic guard, thus avoiding the backflow of the substrate and the exiting of the biogas from the feeding line.

. As an alternative to the accumulating tank (bunker), certain alternative solutions provide the organic waste to be accumulated prior to the pre-treatment in unloading pits from which an automated feeding system feeds the treatment line, by means of an overhead crane provided with a polyp bivalve bucket, at night and during the weekend in the absence of personnel.

. As mentioned above, the passage from the shredder to the sieve or size separator and from the latter to the anaerobic digester is carried out by means of conveyor screws or alternatively, rubber or plate conveyor belts.

**.** A pre-treatment system as the one described above is not devoid of defects.

**.** Firstly, the sifting through holes sized between 50 mm and 80 mm allows the passage into the organic fraction (the sub-sieve) of fragments of plastic, glass, metals, fabrics, mussel shells, cork and plastic corks, coffee pods, and other materials which are not anaerobically digestible, or they are in much longer times than those provided in the process (20-22 days), effectively determining a decrease in the value of the biogas production.

**.** Moreover, the fragments having greater specific weight and greater dimensions not eliminated by the sifting may settle - under certain conditions of substrate viscosity and density - on the bottom of the anaerobic digesters, thus causing breakdowns due to jamming of the movement members and the solenoid valves and digestate unloading pumps.

**.** Therefore, these sediments induce plant shutdowns which are highly damaging in terms of the loss of production and the need for lengthy and laborious maintenance interventions.

**.** It should be noted that in order to decrease the risk of such sediments, it is common to implement the tactic of mixing a significant amount of plant material (for example, ground pruning elements) with the organic fraction, up to and over 15%, which by thickening the substrate, keeps the density increased up to the outlet from the fermenters.

**.** However, given that it is not highly fermentable, this material itself obviously decreases the average biogas production of the mixture.

**.** In addition, it should be noted that the plastic fragments having smaller specific weight (bags, bottles, glasses, etcetera) may trap biogas bubbles and be dragged to the surface in the anaerobic digesters, thus causing the formation of a crust which prevents the biogas from being freed into the head space from which the biogas is usually evacuated and collected.

. This drawback cause a smaller production of biogas and an increase in the level of substrate inside the anaerobic digesters beyond the safety levels.

. Again, the fragments of plastic and other undesired materials which are contained in the substrate exiting the fermenters (digestate) are contaminates for the agricultural use of the digestate as an organic fertilizer.

. Thus, the presence thereof results in the need to subject the solid digestate to complex selection and refining operations.

. Moreover, the average dimensions of the organic waste particles obtained from the pre-treatment are such as to slow down the degradation processes leading to the formation of biogas (hydrolysis, acidogenesis, etcetera) because the microbial attack occurs at the surface of the particles themselves, and therefore the greater the dimension of the particle, the smaller the volume/mass ratio. Indeed, smaller particles have a much greater surface exposed to the microbes per unit of mass.

. Additionally, the organic material obtained from the pre-treatment is a heterogeneous solid at room temperature and therefore, it is difficult to heat up to the desired process temperature. Such a material takes on a fluid consistency which facilitates heat exchange only in the anaerobic digester, when the breakdown processes begin, therefore the heating is obtained directly in the volume of the anaerobic digesters, thus determining, on the inlet side, an area with a temperature which is lower than the optimal temperature, with a reduction in the biogas performance of the process.

. Moreover, the dosing systems of the ingestate by means of the accumulating tank (bunker), and even more by means of automated pits with an overhead crane characterized by several moving machines, having to also operate at night and on non-working days in the absence of personnel, lead to apparent problems related to timeliness of intervention in the case of malfunction.

. Finally, the dosing systems of the ingestate by means of automated pits are affected by the so-called *Last In-First Out* problem, that is, the last organic waste unloaded into the pit is the first to be sent for processing, while the oldest material remains in the pit longer, thus causing a release of percolating liquids which are to be pumped by specific systems and sent to the fermenters or for purification, with apparent plant complications and economic burdens.

### . Summary

. It is the object of the present invention to devise and provide an organic waste pre-treatment system for successive anaerobic digestion which allows the aforesaid drawbacks with reference to the known art to be at least partially obviated, and which in particular, is capable of ensuring increased reliability and efficiency of the anaerobic digester fed with the organic material pre-treated with such a pre-treatment system.

. Such an object is achieved by a system according to claim 1.

. Advantageous embodiments of such a system are the subject of the dependent claims.

. The subject of the present invention is also an organic waste pre-treatment method for successive anaerobic digestion.

### . Brief description of the drawings

. Further features and advantages of the system and related method according to the invention will become apparent from the following description of preferred embodiments, given by way of indicative, non-limiting examples, with reference to the accompanying drawings, in which:
- Figure 1 schematically shows an organic waste pre-treatment system for successive anaerobic digestion according to an embodiment of the present invention;
- Figure 2a schematically shows a side view of a component of the organic waste pre-treatment system for successive anaerobic digestion following the present invention, according to an embodiment of the invention;
- Figure 2b schematically shows a top view of the component of Figure 2a;
- Figure 3a schematically shows a side view of a component of the organic waste pre-treatment system for successive anaerobic digestion following the present invention, according to an embodiment of the invention;
- Figure 3b schematically shows a top view of the component of Figure 3a;
- Figure 4a schematically shows a sectional side view of a component of the organic waste pre-treatment system for successive anaerobic digestion following the present invention, according to an embodiment of the invention;
- Figure 4b schematically shows a top view of the component of Figure 4a;
- Figure 5a schematically shows a sectional side view of a component of the organic waste pre-treatment system for successive anaerobic digestion following the present invention, according to an embodiment of the invention;
- Figure 5b schematically shows a top sectional view of the component of Figure 5a, and
- Figure 6 shows a block diagram of an organic waste pre-treatment method for successive anaerobic digestion according to an embodiment of the present invention.

. It is worth noting that equal or similar elements in the drawings will be indicated by the same numeric or alphanumeric numerals.

### . Description of some preferred embodiments

. Referring now to the aforesaid drawings, numeral 100 indicates an organic waste pre-treatment system for successive anaerobic digestion as a whole, hereinafter also pre-treatment system or simply system.

. The system 100 comprises a first organic waste pre-treatment station 10.

. The first organic waste pre-treatment station 10 is configured to open packages of organic waste and shred the organic waste into particles.

. Moreover, the first organic waste pre-treatment station 10 is configured to convey the organic waste particles to successive processing.

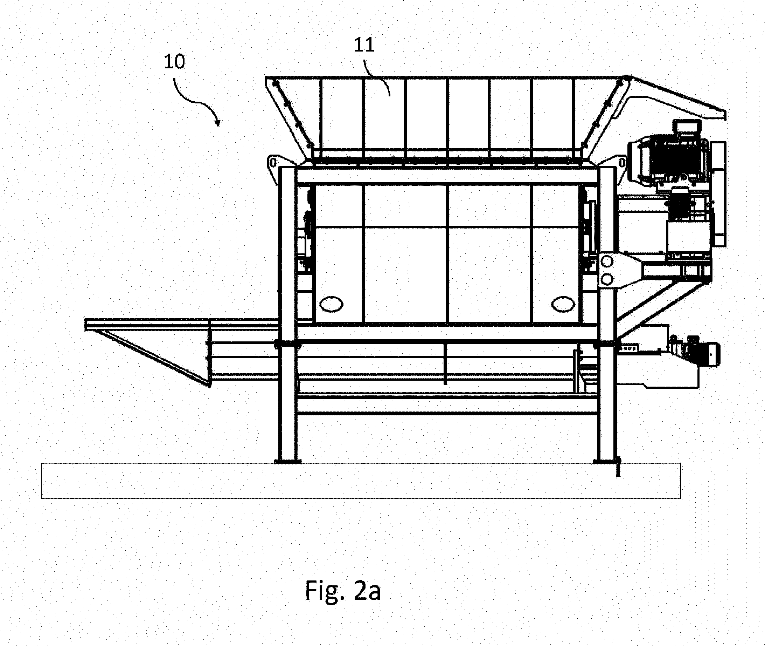

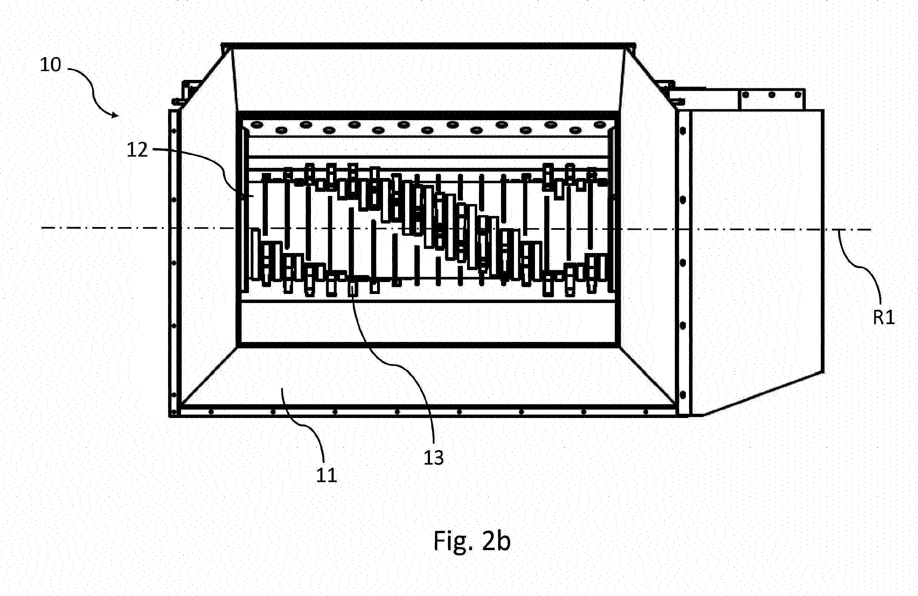

. In an embodiment shown in Figures 2a and 2b, the first organic waste pre-treatment station 10 comprises a tank 11, preferably made of metal material, open at the top to facilitate the introduction of packages (for example, sacks, bags, etcetera) containing organic waste.

. In this embodiment, the first organic waste pre-treatment station 10 further comprises a cylindrical member 12 (for example, a rotor) accommodated inside tank 11, preferably at the bottom of tank 11, and adapted to rotate about a respective rotation axis R1.

**.** The cylindrical member 12 comprises a plurality of metal teeth 13 distributed over the outer surface of the cylindrical member 12.

**.** In this embodiment, the first organic waste pre-treatment station 10 further comprises at least one tearing element (not shown in the drawings), for example, a comb, fastened inside tank 11 at a set distance from the cylindrical member 12.

**.** During the rotation of the cylindrical member 12 about the respective rotation axis R1, the plurality of metal teeth 13 bring the packages of organic waste against the at least one tearing element, thus allowing the latter to open the packages of organic waste and shred the organic waste into particles.

**.** Moreover, the set distance between the cylindrical member 12 and the at least one tearing element preferably allows the passage below tank 11 for conveying to the successive processing only organic waste particles having a smaller size than said set distance.

**.** It should be noted that the set distance between the cylindrical member 12 and the at least one tearing element is such to ensure a non-forced fragmentation of the packages, or of bags and other plastic material in general present in the packages, to allow components (hereinbelow described) of the system 100 arranged downstream of the first organic waste pre-treatment station 10 to operate efficiently.

**.** Not performing a forced fragmentation advantageously allows obtaining improved cleaning of the substrate in the successive pre-treatment station (hereinbelow described) and consequently, of the fertilizer obtained from minute plastic fragments (microplastics) and also less consumption of electrical energy for the lower power of the motors required only for opening the packages.

. It should be noted that the first pre-treatment station 10 is representative of a type of package or bag opening machine.

. Returning in general to the system 100 of the invention, the system 100 further comprises at least a second organic waste pre-treatment station 20 configured to receive organic waste particles conveyed from the first organic waste pre-treatment station 10.

. The at least a second organic waste pre-treatment station 20 is configured to separate such organic waste particles into sub-particles of the moist putrescible organic component and into sub-particles of the non-putrescible waste material component.

. Advantageously, the at least a second organic waste pre-treatment station 20 is configured to convey a compound of sub-particles of the moist putrescible organic component having a smaller size than a set sifting size, to a successive processing.

. The set sifting size is less than or equal to 40 mm, preferably less than or equal to 30 mm.

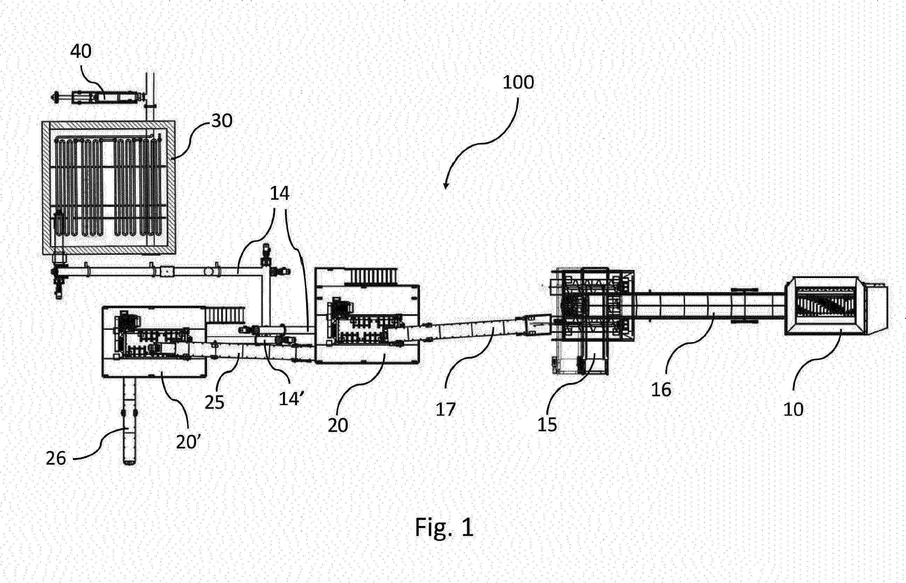

. The conveying of the compound of sub-particles of the moist putrescible organic component having a smaller size than the set sifting size, to the successive processing occurs by means of one or more motorized transport members 14 (shown in Figure 1), for example one or more conveyor screws arranged downstream of the at least a second organic waste pre-treatment station 20.

**.** According to an embodiment shown in Figure 1, the system 100 further comprises an accumulating and dosing hopper 15 of organic waste particles coming from the first organic waste pre-treatment station 10.

. It should be noted that the conveying of particles from the first organic waste pre-treatment station 10 and the accumulating and dosing hopper 15 of particles occurs by means of a first motorized loading member 16, for example a conveyor screw (shown in Figure 1).

. The accumulating and dosing hopper 15 of organic waste particles conveyed from the first organic waste pre-treatment station 10 is configured to convey the accumulated organic waste particles towards the at least a second organic waste pre-treatment station 20.

. In this regard, the conveying of the organic waste particles from the accumulating and dosing hopper 15 of organic waste particles to the at least a second organic waste pre-treatment station 20 occurs by a second motorized loading member 17, for example a conveyor screw (shown in Figure 1).

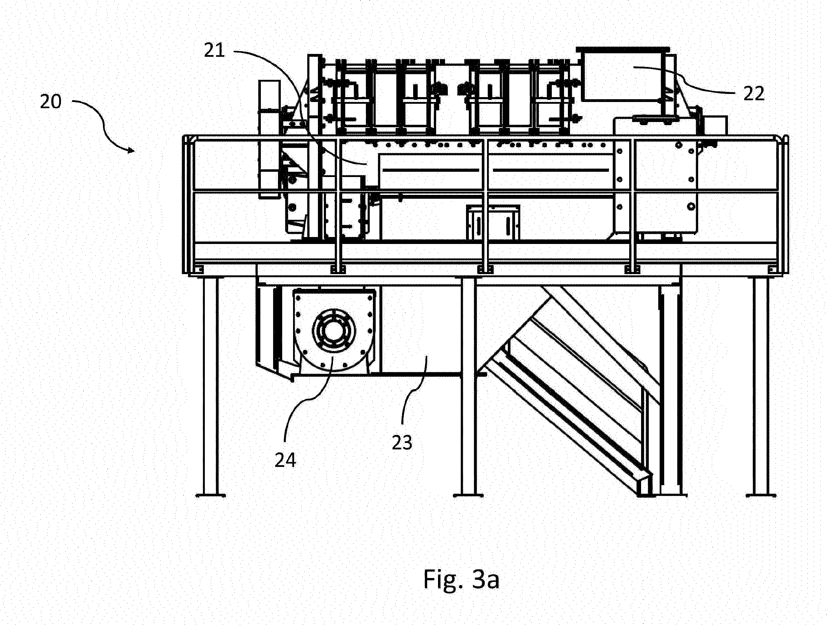

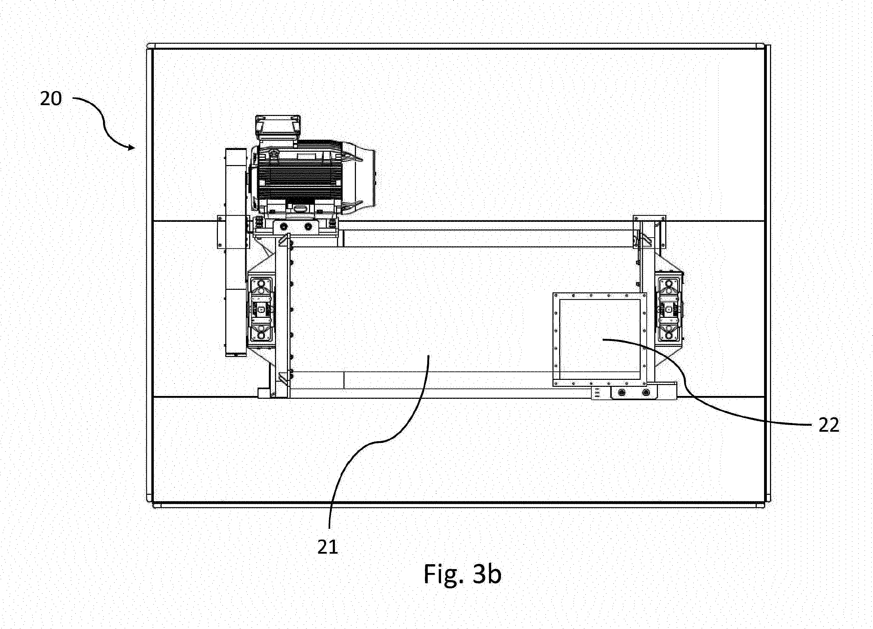

. In an embodiment shown in Figures 3a and 3b, the at least a second organic waste pre-treatment station 20 comprises a metal cylinder 21 having horizontal axis, where a rotating shaft rotates, on which rotating blades or idle hammers with replaceable head are mounted.

. The at least a second station 20 comprises an inlet hopper 22 placed at an end of the metal cylinder 21 and above the same. The inlet hopper 20 is adapted to feed the organic waste particles coming from the first organic waste pre-treatment station 10.

. The organic waste particles coming from the first organic waste pre-treatment station 10 are fed by means of the inlet hopper 22 placed at an end of the metal cylinder 21 and above the same.

**.** The at least a second organic waste pre-treatment station 20 comprises an unloading hopper 23 placed below the metal cylinder 21, the rotating blades of the rotating shaft being adapted, when the rotating shaft is put into rotation conventionally at a frequency in the order of some hundreds of revolutions per minute, to hit such particles to be treated, thus causing the forced disintegration of the sub-particles of the moist putrescible organic component (with smaller size than the set sifting size), pushing such sub-particles towards the unloading hopper 23 (shown in Figure 3a) and the sub-particles of the non-putrescible waste material component (such as plastics, metals, fabrics and the like, having larger size than the set sifting size) to advance in axial direction in the cavity towards a respective drain 24 (shown in Figure 3a) placed at the lower end opposite to the inlet hopper 22.

**.** When the rotating shaft is put into rotation, conventionally at a frequency in the order of some hundreds of revolutions per minute, the rotating blades hit such particles to be treated, thus causing the forced disintegration of the sub-particles of the moist putrescible organic component (with smaller size than the set sifting size), pushing such sub-particles towards the unloading hopper 23 (shown in Figure 3a) and the sub-particles of the non-putrescible waste material component (such as plastics, metals, fabrics and the like, having larger size than the set sifting size) to advance in axial direction in the cavity towards the respective drain 24 (shown in Figure 3a) placed at the lower end opposite to the inlet hopper 22.

**.** The compound of sub-particles of the moist putrescible organic component (puree) instead is exiting through a fixed sifting net with holes having diameter not greater than the set sifting size, for example equal to 30 mm, forming the lower part of the cylinder.

. The compound of sub-particles of the moist putrescible organic component having a smaller size than the set sifting size is adapted to fall below a net into the unloading hopper 23 placed below the metal cylinder 21 and from there it is adapted to be removed and sent to the next step by means of the one or more motorized transport members 14.

. The compound of sub-particles of the moist putrescible organic component having a smaller size than the set sifting size falls below a net into the unloading hopper 23 placed below the metal cylinder 21 and from there, it is removed and sent to the next step by means of one or more motorized transport members 14.

. It should be noted that the at least a second organic waste pre-treatment station 20 according to the embodiment in Figures 3a and 3b, is of the quick separator type.

. It is worth noting that while the separators belonging to the prior art and available on the market usually provide the addition of water to facilitate the separation of the putrescible organic fraction from the undesired materials and plastics in particular, according to the above embodiment, it is possible not to provide for the use of liquids, except for minimum amounts for the cleaning at shift end, in order not to dilute the substrate and remain within a DRY environment and benefit from the related advantages described above.

. This decreases the separation efficiency of the organic fraction with respect to use with diluting water.

. However, according to an embodiment of the invention shown in Figure 1, the system 100 further comprises a further second organic waste pre-treatment station 20', similar to the at least a second organic waste pre-treatment station 20 described with reference to the embodiment of Figures 3a and 3b and other embodiments.

**.** The further second organic waste pre-treatment station 20' is configured to subject the material discarded from the treatment of the at least a second organic waste pre-treatment station 20, that is the sub-particles of the component of non-putrescible waste material, to a second treatment so as to reduce the amount of moist organic material remaining attached to the non-organic components and obtain an increased amount of putrescible organic waste to be sent to anaerobic digestion.

**.** The conveying of the material discarded from the treatment performed by the at least a second organic waste pre-treatment station 20 to the further second organic waste pre-treatment station 20' occurs by means of a further motorized loading member 25 (shown in Figure 1), for example a conveyor screw.

**.** The undesired waste obtained from the processing by the further second organic waste pre-treatment station 20' is predominantly represented by plastics and inert materials and may thus be sent to disposal or possible recovery in another plant.

**.** As shown in Figure 1, sending out for disposal or possible recovery in another plant occurs by means of a further motorized unloading member 26 (for example, a conveyor screw).

**.** Instead, the sub-particles of the moist putrescible organic component are removed and sent to the successive step by means of one or more motorized transport members 14' (shown in Figure 1), for example one or more conveyor screws, arranged downstream of the further second organic waste pre-treatment station 20'.

. According to a further embodiment not shown in the drawings, alternatively to the one shown in Figures 1, 3a and 3b, the at least a second organic waste pre-treatment station 20 comprises a perforated cylindrical chamber with holes having smaller diameter than the set second sifting size, for example less than or equal to 40 mm, preferably less than or equal to 30 mm.

. In this embodiment, the conveying of the organic waste particles from the accumulating and dosing hopper 15 of particles, coming from the first organic waste pre-treatment station 10, to the at least a second organic waste pre-treatment station 20, occurs by means of a push by a conveyor screw or feed-screw having constant or varying pitch, at the end of which an adjustment device determines the outlet section, allowing the adjusting of the pressure at which the organic waste is squeezed and accordingly, the separation performance of a moist putrescible organic component into sub-particles and of a component of non-putrescible waste material into sub-particles.

. It is worth noting that the at least a second pre-treatment station 20 of the embodiment described is of the slow squeezing press or bio-extruder type.

. According to an embodiment in combination with the previous one, the system 100 may comprise the further second organic waste pre-treatment station 20' for a double passage processing to increase the separation performance and minimize the amount of waste according to the needs of the operator.

. Several advantages are obtained with both the above-described embodiments of said at least a second organic waste pre-treatment station 20.

**.** Firstly, the compound (puree) of sub-particles of the moist putrescible organic component having a smaller size than the set sifting size does not contain inert and non-digestible materials having greater sizes than the set sifting size (for example, 30 mm), unlike the sub-sieve which may contain fragments up to 50-80 mm in diameter.

**.** Thus, the subsequent risk of sedimentation in the digesters is minimized.

**.** Indeed, the sedimentation speed is a square function of the radius of the particle [Stokes law - a particle having average radius of 30 mm (sub-sieve) will sediment at four times the speed with respect to a particle of the same material but with an average radius of 15 mm (puree)].

**.** Moreover, the joint effect of the first organic waste pre-treatment station 10 (bag opener) used which does not tear the plastic bags and the separating machines with reduced holes, results in a highly reduced content of plastic fragments and a subsequent minimum microplastic contamination of the digestate and the compost obtained.

**.** The compound (puree) obtained is viscous but pumpable and therefore may be transferred to the successive steps by means of hydraulic systems such as pumps and conveyor screws, thus simplifying the management and maintenance of the equipment very much.

**.** Such a compound (puree) appears as a viscous mud and perfectly amalgamated with dry substance content conventionally greater than 25% (measured in the absence of cuttings) which is equal to the one of the organic waste from which it is obtained because no water is added during the treatment.

**.** The size of the particles forming the mud is not greater than 40 mm, and preferably not greater than 30 mm.

. The material with these features is pumpable and therefore fed by means of a piston pump (as described later).

. Returning in general to the invention in Figure 1, the system 100 further comprises a third organic waste pre-treatment station 30 configured to accumulate the compound of sub-particles of the moist putrescible organic component having a smaller size than the set sifting size.

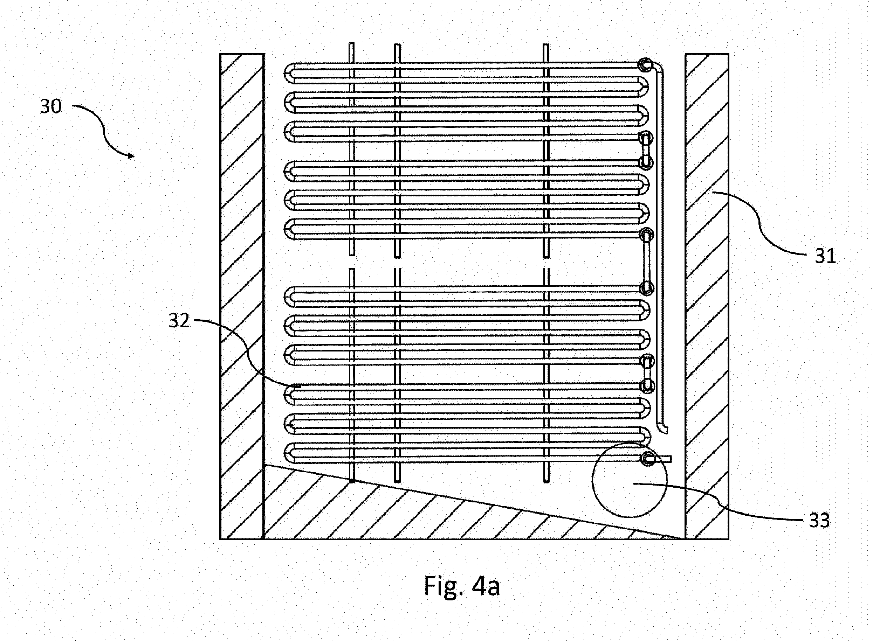

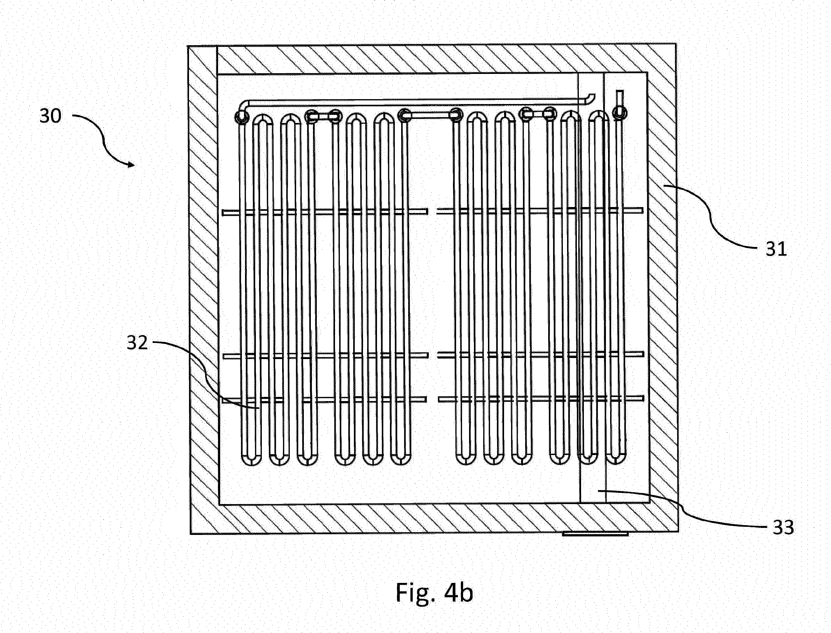

. According to an embodiment shown in Figures 4a and 4b, the third organic waste pre-treatment station 30 comprises an accumulating and feeding tank 31.

. Such a tank 31 preferably is made of reinforced concrete, therefore is impermeable to liquids.

. Moreover, tank 31 has an inner volume which is adapted to a continuous feeding of anaerobic digesters also on weekend days or non-working days.

. The bottom of tank 31 preferably is inclined, as shown for example in Figure 4a.

. In an embodiment, in combination with the preceding one, tank 31 comprises a heating apparatus 32 configured to heat the compound at a set temperature close to the process temperature.

. According to an embodiment, the heating apparatus 32 is a heat exchanger comprising one or more coils heated with hot water which are located on the inner walls and/or bottom of tank 31.

. With reference in general to the embodiment in Figures 4a and 4b, the third organic waste pre-treatment station 30 further comprises a further motorized unloading member 33 (for example, a conveyor screw) attached to the inclined bottom of tank 31.

**.** The motorized unloading member 33 is configured to slowly facilitate the removal of the compound from tank 31 to convey it towards the anaerobic digesters.

**.** With of the employment of the third organic waste pre-treatment station 30 described above according to various embodiments, by mixing with the substrate content, the organic compound advantageously is preliminarily and gradually heated so that the inlet thereof into the anaerobic digester is not subjected to and does not cause thermal shock to the microbial community.

**.** Thus, the volume inside the fermenters is used in optimal manner.

**.** Moreover, staying in the tank after disintegration and the preheating promote the immediate establishment of hydrolysis and acidogenesis deteriorating processes and shorten the start of the biogas production (acetogenesis and methanogenesis) which are performed in the successive step in the fermenters.

**.** Again, unlike the feeding and dosing bunkers with movable plate bottom, the reinforced concrete tank is impermeable to liquids; moreover, the disintegrating action of the centrifugal separators results in the formation of a high density and viscosity compound (puree) which, due to the colloidal nature thereof, does not promote the release of liquids, and accordingly there are no problems of percolates leaking from the bunker and requiring continuous collection interventions and cleaning of the rooms where they are installed.

**.** Finally, unlike the systems implementing supply pits for the preliminary storing and in which the processing with all the pre-treatment machines continuously occurs throughout the entire day, including non-working days and therefore in the absence of personnel, the implementation of accumulating and feeding tanks and the mechanical components involved in this section are minimized (facilitating conveyor screw), and accordingly the risks of malfunction/breakage in the absence of personnel are also minimized.

**.** Returning in general to the invention in Figure 1, the system 100 further comprises a feeding device 40 configured to transfer the compound, eventually heated, from the third organic waste pre-treatment station 30 (accumulating and feeding tank 31) to anaerobic digesters (not shown in the drawings).

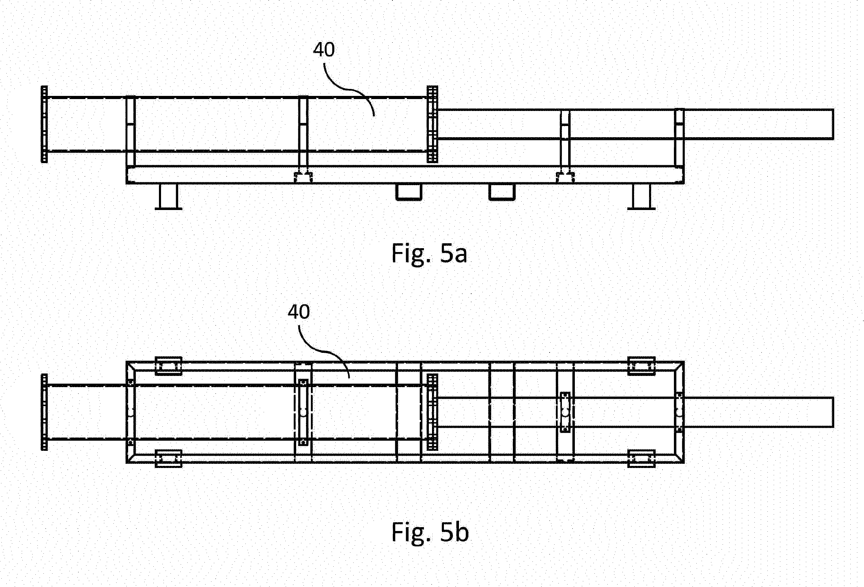

**.** According to an embodiment, in combination with any one of the ones described above shown in Figures 1, 5a and 5b, the feeding device 40 comprises a hydraulic piston pump for high viscosity materials.

**.** A feeding pump of this type is commonly employed to empty and recirculate the anaerobic digesters of the digestate, the latter being fluid and pumpable, while they usually are not employed for the ingestate because it is in the inhomogeneous solid and non-pumpable state.

**.** However, according to the present invention, the possibly preheated compound of sub-particles of the moist putrescible organic component having a smaller size than the set sifting size (not greater than 40 mm) is pumpable, therefore also the employment of the hydraulic piston pump for transferring the ingestate advantageously is promoted.

**.** The possibility of implementing one or more hydraulic piston pumps for loading the anaerobic digesters allows extreme simplification of the layout of the system 100 by, for example, eliminating the employment of hoppers, belts and inclined conveyor screws, with apparent maintenance advantages and fewer risks of plant shutdown.

. Moreover, a hydraulic piston pump is of the volumetric type and thus allows an accurate volumetric measuring of the amounts introduced into the process, unlike the conventional systems based on the employment of dynamic loading cells which are less accurate and reliable.

. Additionally, a hydraulic piston pump advantageously prevents the backflow of the ingestate also if introduced below the hydraulic doors, and therefore there is no need to bring the inlet pipes above the filling level of the digesters (usually at about 6-8 meters high).

. According to other embodiments not shown in the drawings, alternatively to the preceding one, the feeding device 40 may be a motorized loading member, for example a conveyor screw.

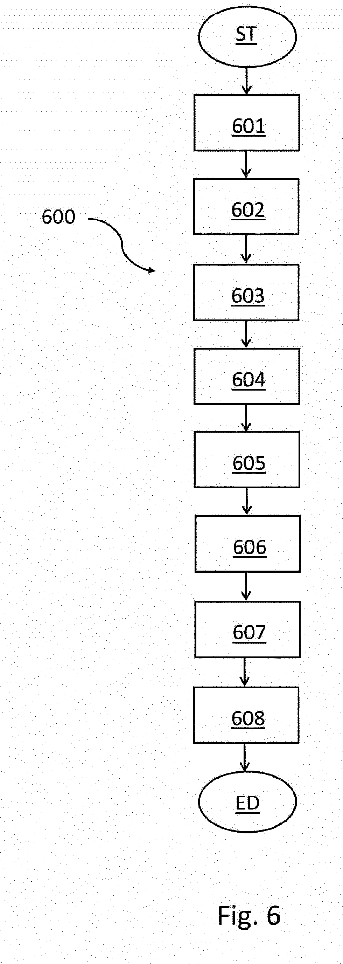

. Referring now to Figure 6, a pre-treatment method 600 of organic waste for successive anaerobic digestion, later also pre-treatment method or simply method, is now described.

. The method 600 comprises a symbolic starting step ST.

. The method 600 further comprises a step 601 of opening, by a first organic waste pre-treatment station 10, packages of organic waste.

. The method 600 further comprises a step 602 of shredding, by the first organic waste pre-treatment station 10, organic waste into particles.

. The method 600 further comprises a step 603 of conveying to a successive processing, by the first organic waste pre-treatment station 10, organic waste particles.

**.** The first organic waste pre-treatment station 10 was described above with reference to the system 100.

. The method 600 further comprises a step 604 of receiving, by at least a second organic waste pre-treatment station 20, the organic waste particles conveyed from the first organic waste pre-treatment station 10.

. The method 600 further comprises a step 605 of separating, by the at least a second organic waste pre-treatment station 20, such particles into sub-particles of a moist putrescible organic component and into sub-particles of a non-putrescible waste material component.

. The method 600 further comprises a step 606 of conveying to a successive processing, by the at least a second organic waste pre-treatment station 20, a compound of sub-particles of the moist putrescible organic component having a smaller size than a set sifting size.

. According to various embodiments, the at least a second organic waste pre-treatment station 20 and the set sifting size were described above with reference to the system 100.

. The method 600 further comprises a step 607 of accumulating, by a third organic waste pre-treatment station 30, the compound of sub-particles of the moist putrescible organic component having smaller size than the set sifting size.

. The third organic waste pre-treatment station 30 was described above.

. The method 600 further comprises a step 608 of transferring the compound, by a feeding device 40, from the third organic waste pre-treatment station 30 (accumulating and feeding tank 31) to anaerobic digesters (not shown in the drawings).

. The feeding device 40 according to various embodiments was described above.

. According to an embodiment, the transfer step 608 is carried out by employing a hydraulic piston pump for high viscosity materials as feeding device 40 (described above).

. The method 600 comprises a symbolic step of ending ED.

. Referring now to the drawings, an operating example of the organic waste pre-treatment system 100 for successive anaerobic digestion according to an embodiment of the present invention, is now described.

. A first organic waste pre-treatment station 10 (package opening machine) opens the packages of organic waste accommodated therein and shreds the organic waste into particles.

. The first organic waste pre-treatment station 10 conveys the organic waste particles to a successive processing.

. At least a second organic waste pre-treatment station 20 (quick separator or slow squeezing press) receives, from an accumulating and dosing hopper 15, organic waste particles coming from the first organic waste pre-treatment station 10 and separates such particles into sub-particles of a moist putrescible organic component and into sub-particles of a non-putrescible waste material component.

. The at least a second organic waste pre-treatment station 20 conveys a compound of sub-particles of the moist putrescible organic component having a smaller size than a set sifting size to a successive processing.

. A third organic waste pre-treatment station 30 (accumulating and feeding tank) accumulates and preheats the compound of sub-particles of the moist putrescible organic component having smaller size than the set sifting size.

**.** A feeding device 40 (for example, a hydraulic piston pump for high viscosity materials) transfers the preheated compound from the third organic waste pre-treatment station 30 (accumulating and feeding tank) to anaerobic digesters.

**.** It is apparent that the object of the present invention is completely achieved because the system and the related method object of the present invention have the advantages described above.

**.** Those skilled in the art may make changes and adaptations to the embodiments of the system and related method described above or can replace elements with others which are functionally equivalent in order to satisfy contingent needs without departing from the scope of protection of the appended claims. All the features described above as belonging to a possible embodiment can be implemented independently of the other embodiments described.

## Claims

1. An organic waste pre-treatment system (100) for successive anaerobic digestion, comprising:
- a first organic waste pre-treatment station (10) configured to open packages of organic waste and shred the organic waste into particles, said first organic waste pre-treatment station (10) being configured to convey the waste particles to a successive processing;
- at least a second organic waste pre-treatment station (20) configured to receive the organic waste particles conveyed from the first organic waste pre-treatment station (10), said at least a second organic waste pre-treatment station (20) being configured to separate such particles into sub-particles of a moist putrescible organic component and into sub-particles of a non-putrescible waste material component, said at least a second organic waste pre-treatment station (20) being configured to convey a compound of sub-particles of the moist putrescible organic component having a smaller size than a set sifting size to a successive processing;
- a third pre-treatment station (30) configured to accumulate the compound of sub-particles of the moist putrescible organic component having a smaller size than a set sifting size;
- a feeding device (40) configured to transfer the compound from the third organic waste pre-treatment station (30) to anaerobic digesters.

2. A system (100) according to claim 1, further comprising an accumulating and dosing hopper (15) of organic waste particles coming from the first organic waste pre-treatment station (10), the accumulating and dosing hopper (15) of organic waste particles conveyed from the first organic waste pre-treatment station (10) being configured to convey the accumulated organic waste particles towards the at least a second organic waste pre-treatment station (20).

3. A system (100) according to any one of the preceding claims, wherein the first organic waste pre-treatment station (10) comprises:
- a tank (11) which is open at the top to promote the insertion of packages of organic waste;
- a cylindrical member (12) accommodated in the tank (11) and adapted to rotate about a respective rotation axis (R1), the cylindrical member (12) comprising a plurality of metal teeth (13) distributed over the outer surface of the cylindrical member (12);
- at least one tearing element fastened to the inside of the tank (11) at a set distance from the cylindrical member (12), during the rotation of the cylindrical member (12) about the respective rotation axis (R1) the plurality of metal teeth (13) bring the packages of organic waste against the at least one tearing element, thus allowing the latter to open the organic waste packages and shred the organic waste into particles.

4. A system (100) according to any one of the preceding claims, wherein the at least a second organic waste pre-treatment station (20) comprises a metal cylinder (21) having horizontal axis about which a rotating shaft rotates, on which rotating blades or idle hammers with replaceable head are mounted, the at least a second station (20) comprising an inlet hopper (22) placed at an end of the metal cylinder (21) and above the same, said inlet hopper (22) being adapted to feed the organic waste particles coming from the first organic waste pre-treatment station (10), the at least a second organic waste pre-treatment station (20) comprising an unloading hopper (23) placed below the metal cylinder (21), the rotating blades of the rotating shaft being adapted, when the rotating shaft is put into rotation, to hit such particles to be treated, thus causing the forced disintegration of the sub-particles of the moist putrescible organic component by pushing such sub-particles towards the unloading hopper (23) and the sub-particles of the non-putrescible waste material component to advance in axial direction in the cavity towards a respective drain (24) placed at the opposite lower end with respect to the inlet hopper (22), the compound of sub-particles of the moist putrescible organic component having a smaller size than the set sifting size, thus falling below a net into the unloading hopper (23) placed below the metal cylinder (21) and from there, being able to be removed by means of one or more motorized transport members (18) and sent to the next step.

5. A system (100) according to any one of preceding claims 1 to 3, wherein the at least a second organic waste pre-treatment station (20) comprises a perforated cylindrical chamber with holes having smaller diameter than the set sifting size.

6. A system (100) according to any one of the preceding claims, further comprising a further second organic waste pre-treatment station (20') configured to subject the material discarded from the treatment of the at least a second organic waste pre-treatment station (20) to a second treatment so as to reduce the amount of moist organic material remaining attached to the non-organic components and obtain an increased amount of putrescible organic waste to be sent to anaerobic digestion.

7. A system (100) according to any one of the preceding claims, wherein the third organic waste pre-treatment station (30) comprises an accumulating and feeding tank (31).

8. A system (100) according to claim 6, wherein the tank (31) is made of reinforced concrete.

9. A system (100) according to either of preceding claims 7 or 8, wherein the tank (31) comprises a heating apparatus (32) configured to heat the compound at a set temperature close to the process temperature.

10. A system (100) according to any one of preceding claims 7 to 9, wherein the third organic waste pre-treatment station (30) further comprises a further motorized unloading member (33) attached to the inclined bottom of the tank (31), the motorized unloading member (33) being configured to slowly facilitate the removal of the compound from the tank (31) to convey it to anaerobic digesters.

11. A system (100) according to any one of the preceding claims, wherein the feeding device (40) comprises a hydraulic piston pump for high viscosity materials.

12. A system (100) according to any one of the preceding claims, wherein the set sifting size is less than or equal to 40 mm.

13. A system (100) according to any one of preceding claims 1 to 11, wherein the set sifting size is less than or equal to 30 mm.

14. A method (600) for pre-treating organic waste for successive anaerobic digestion, comprising steps of:
- opening (601), by a first organic waste pre-treatment station (10), packages of organic waste;
- shredding (602), by the first organic waste pre-treatment station (10), the organic waste into particles;
- conveying (603) to a successive processing, by the first organic waste pre-treatment station (10), organic waste particles;
- receiving (604), by at least a second organic waste pre-treatment station (20), the organic waste particles conveyed from the first organic waste pre-treatment station (10);
- separating (605), by said at least a second organic waste pre-treatment station (20), such particles into sub-particles of a moist putrescible organic component and into sub-particles of a non-putrescible waste material component;
- conveying (606) to a successive processing, by the at least a second organic waste pre-treatment station (20), a compound of sub-particles of the moist putrescible organic component having a smaller size than a set sifting size;
- accumulating (607), by a third organic waste pre-treatment station (30), the compound of sub-particles of the moist putrescible organic component having smaller size than the set sifting size;
- transferring (608) the compound, by a feeding device (40), from the third organic waste pre-treatment station (30) to anaerobic digesters.

15. A method (600) according to claim 14, wherein the step of transferring (608) is carried out by employing a hydraulic piston pump for high viscosity materials as feeding device (40).
